(19) Europäisches Patentamt
European Patent Office
Office européen des brevets

(11) Veröffentlichungsnummer: **0 021 339**
**B1**

(12) # EUROPÄISCHE PATENTSCHRIFT

(45) Veröffentlichungstag der Patentschrift: **09.02.83**

(21) Anmeldenummer: **80103397.8**

(22) Anmeldetag: **18.06.80**

(51) Int. Cl.³: **C 07 H 15/18,** C 07 H 13/08, A 61 K 31/70 // C07C63/66, C07C51/09, C07C51/60, C07C49/792, C07C45/46, C07C33/34, C07C29/136, C07C21/24, C07C17/16, C07C69/76, C07C67/343

(54) **Zuckerester und Glykoside, deren Herstellung und diese Stoffe enthaltende pharmazeutische Präparate.**

(30) Priorität: **21.06.79 CH 5808/79**

(43) Veröffentlichungstag der Anmeldung: **07.01.81 Patentblatt 81/1**

(45) Bekanntmachung des Hinweises auf die Patenterteilung: **09.02.83 Patentblatt 83/6**

(84) Benannte Vertragsstaaten: **AT BE CH DE FR GB IT LI LU NL SE**

(56) Entgegenhaltungen:
**EP-A-0 002 742**
**DE-A-2 819 213**
**FR-A-2 141 532**

(73) Patentinhaber: **F. HOFFMANN-LA ROCHE & CO. Aktiengesellschaft, CH-4002 Basel (CH)**

(72) Erfinder: **Bollag, Werner, Dr., Mythenstrasse 10, CH-4054 Basel (CH)**
Erfinder: **Wyss, Pierre-Charles, Dr., Unterwartweg 27, CH-4132 Muttenz (CH)**

(74) Vertreter: **Lederer, Franz, Dr. et al, Patentanwälte Dr. Franz Lederer Reiner F. Meyer Lucile-Grahn-Strasse 22, D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 021 339 B1

BUNDESDRUCKEREI BERLIN

Zuckerester und Glykoside, deren Herstellung und diese Stoffe enthaltende pharmazeutische Präparate

Die Erfindung betrifft neue Zuckerester und Glykoside der Formel

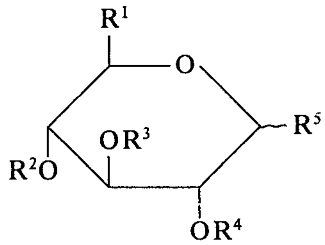

I

worin
$R^1$ ein Rest $-CH_2OH$, $-CH_2OR^6$, $-COR^7$ oder $-CONH_2$;
$R^2$ Wasserstoff oder Acetyl;
$R^3$ und $R^4$ Wasserstoff, Acetyl oder ein Rest $R^6$;
$R^5$ $C_{1-7}$-Alkoxy, ein Rest $OR^6$ oder $OR^8$;
$R^6$ ein Rest der Formel

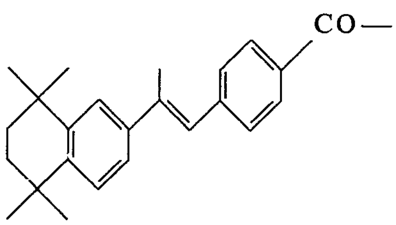

$R^7$ Hydroxy, $C_{1-7}$-Alkoxy oder OM und M ein Kation; und
$R^8$ ein Rest der Formel

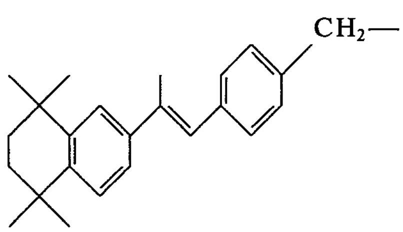

ist,
wobei $R^1$ ein Rest $CH_2OR^6$, $R^2$ Wasserstoff und einer der Reste $R^3$ und $R^4$ Wasserstoff und der andere ein Rest $R^6$ ist, falls $R^5$ $C_{1-7}$-Alkoxy ist; und wobei $R^1$ ein Rest $-COR^7$ ist, und $R^2$, $R^3$ und $R^4$ Acetyl sind, falls $R^5$ ein Rest $OR^6$ ist; und wobei $R^1$ ein Rest $-CH_2OH$, $-COR^7$ oder $-CONH_2$ ist und $R^2$, $R^3$ und $R^4$ Wasserstoff oder Acetyl sind, falls $R^5$ ein Rest $OR^8$ ist.

Die Erfindung betrifft weiterhin ein Verfahren zur Herstellung der Verbindungen der Formel I sowie pharmazeutische Kompositionen auf der Basis der Verbindungen der Formel I.

Eine bevorzugte Gruppe unter den erfindungsgemäßen Verbindungen sind die Verbindungen der Formel

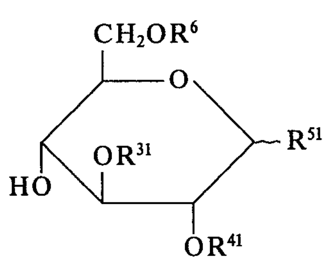

Ia

worin $R^{51}$ $C_{1-7}$-Alkoxy und einer der Reste $R^{31}$ und $R^{41}$ ein Rest $R^6$ und der andere Wasserstoff ist und $R^6$ die oben angegebene Bedeutung besitzt.

Von besonderem Interesse sind die Verbindungen der Formel Ia, in denen $R^{51}$ Methoxy, insbesondere $\alpha$-Methoxy ist.

Eine weitere, bevorzugte Verbindungsgruppe sind die Verbindungen der Formel

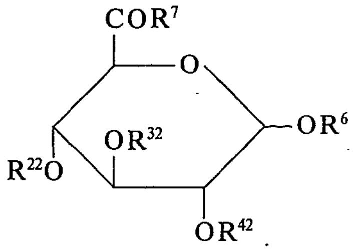

Ib

worin $R^{22}$, $R^{32}$ und $R^{42}$ Acetyl sind und $R^6$ und $R^7$ die oben angegebene Bedeutung besitzen.

Von diesen Verbindungen sind die Verbindungen mit $R^7$ = Methoxy besonders bevorzugt.

Ein weiterer Aspekt der vorliegenden Erfindung betrifft die Verbindungen der Formel

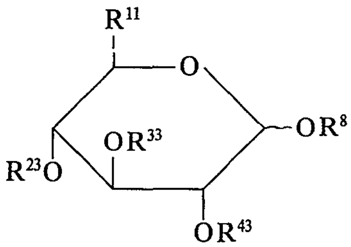

Ic

worin $R^{11}$ ein Rest $CH_2OH$, $COR^7$ oder $CONH_2$; und $R^{23}$, $R^{33}$, $R^{43}$ Wasserstoff oder Acetyl sind und $R^8$ die oben angegebene Bedeutung besitzt..

Von den Verbindungen der Formel Ic sind diejenigen mit $R^{11} = -CONH_2$ oder $-COONa$ und $R^{23}$, $R^{33}$ und $R^{43}$ = Wasserstoff besonders bevorzugt.

Beispiele von $C_{1-7}$ Alkoxygruppen sind Methoxy, Äthoxy, Propoxy und Butoxy. Ein im Rest $R^7$ enthaltenes Kation ist vorzugsweise ein Alkalimetallkation, insbesondere $Na^+$.

Die Verbindungen der Formel I können erfindungsgemäß dadurch erhalten werden, daß man

a) eine Verbindung der Formel

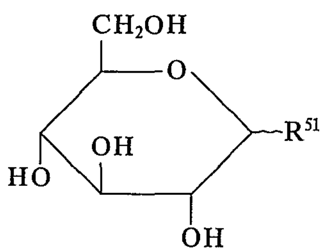

II

oder

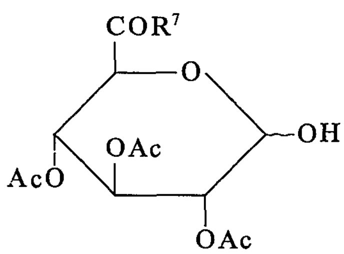

II-1

worin $R^{51}$ $C_{1-7}$-Alkoxy bedeutet,
mit der Säure $R^6OH$ oder einem reaktionsfähigen Derivat davon in Gegenwart eines wasser- bzw. säurebindenden Mittels umsetzt, oder

b) eine Verbindung der Formel

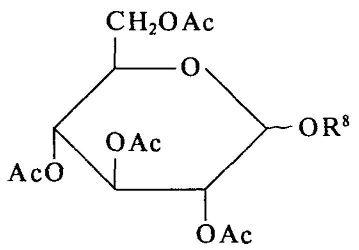

III

worin Ac Acetyl bedeutet und $R^8$ die oben angegebene Bedeutung hat, deacetyliert, oder

c) eine Verbindung der Formel

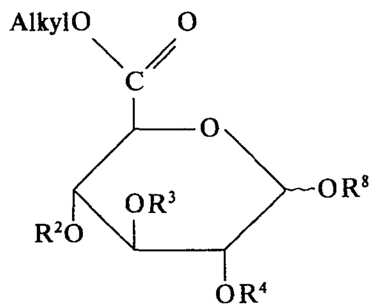

IV

mit methanolischem Ammoniak behandelt, und gewünschtenfalls eine erhaltene Verbindung der Formel I, in der $R^1$ $C_{1-7}$-Alkoxycarbonyl ist, zu einer Verbindung, in der $R^1$ Carboxyl ist, oder in ein Salz einer solchen Verbindung überführt.

Als reaktionsfähiges Derivat der Säure $R^6OH$ kommen insbesondere die Halogenide, z. B. das Chlorid, in Betracht. Die Umsetzung gemäß Verfahrensvariante a) erfolgt zweckmäßig in einem inerten Lösungsmittel. Beispiele von Lösungsmitteln sind Äther, z. B. Diäthyläther, Tetrahydrofuran oder Dioxan; oder Wasser; oder Gemische davon. Beispiele für bei dieser Umsetzung geeignete Basen sind organische Basen wie Pyridin, Homologe davon und anorganische Basen wie Alkalimetallcarbonate. Die Umsetzung wird zweckmäßig bei Temperaturen von 0° bis Zimmertemperatur durchgeführt.

Die Deacetylierung gemäß Verfahrensvariante b) kann durch Behandlung mit Basen, wie Alkalimetallalkoxiden, z. B. Natriummethoxid in Methanol, bewerkstelligt werden. Diese Reaktion kann bei Raumtemperatur durchgeführt werden.

Die Umsetzung gemäß Verfahrensvariante c) kann durch Behandlung einer Verbindung der Formel IV mit methanolischem Ammoniak bei Raumtemperatur durchgeführt werden.

Die Umwandlung einer Alkoxycarbonylgruppe $R^1$ in einer Verbindung der Formel I in die Carboxylgruppe bzw. eine Carboxylatgruppe kann durch Verseifung, z. B. mit Alkali wie alkoholischen Alkalihydroxiden, bewerkstelligt werden. Die so erhaltenen Salze können durch Neutralisation, z. B. mit Kationenaustauschern in der $H^+$-Form, in die freien Säuren übergeführt werden.

Die Ausgangsstoffe können wie in den Beispielen beschrieben oder in Analogie dazu hergestellt werden.

Die Verbindungen der Formel I sind als Wirkstoffe für Pharmazeutika, z. B. zur Behandlung von Neoplasmen, insbesondere zur topischen Verabreichung von Wert. Sie können weiterhin zur Behandlung von Akne und Psoriasis, und zur Behandlung von entzündlichen und allergischen Dermatosen eingesetzt werden. Die Verbindungen zeichnen sich besonders durch eine gute Verträglichkeit, z. B. das Fehlen von Hautirritationen bei der topischen Verabreichung, aus.

Die tumorhemmende Wirkung der Verbindungen wurde an Mäusen geprüft, bei denen durch Behandlung mit Dimethylbenzanthracen und Krotonöl Papillome der Haut erzeugt wurden. Durch Verabreichung von Verbindungen der Formel I wurde eine Rückbildung der Papillome beobachtet. Die Versuchsresultate sind der Tabelle I zu entnehmen.

Tabelle I

| Verbindung | Dosis | Verringerung des Durchmessers der Papillome (o/o) |
|---|---|---|
| A | 0,1 | −22 |
| B | 6 | −33 |
| C | 1,5 | −38 |
| D | 0,75 | −57 |
| E | 0,4 | −69 |
| F | 0,1 | −25 |
| G | 0,1 | −57 |

A: p-[(E)-2-(5,6,7,8-Tetrahydro-5,5,8,8-tetramethyl-2-naphthyl)-propenyl]-benzyl-β-D-glucopyranosid

B: p-[(E)-2-(5,6,7,8-Tetrahydro-5,5,8,8-tetramethyl-2-naphthyl)-propenyl]-benzyl-β-D-glucopyranosid-uronamid

C: Natrium-p-[(E)-2-(5,6,7,8-tetrahydro-5,5,8,8-tetramethyl-2-naphthyl)-propenyl]-benzyl-β-D-glucopyranosid-uronat

D: Methyl-2,6-bis-O-[p-[(E)-2-(5,6,7,8-tetrahydro-5,5,8,8-tetramethyl-2-naphthyl)-propenyl]-benzoyl]-α-D-glucopyranosid

E: Methyl-3,6-bis-O-[p-[(E)-2-(5,6,7,8-tetrahydro-5,5,8,8-tetramethyl-2-naphthyl)-propenyl]-benzoyl]-α-D-glucopyranosid

F: Methyl-2,3,4-tri-O-acetyl-1-O-[p-[(E)-2-(5,6,7,8-tetrahydro-5,5,8,8-tetramethyl-2-naphthyl)-propenyl]-benzoyl]-α-D-glucopyranuronat

G: Methyl-2,3,4-tri-O-acetyl-1-O-[p-[(E)-2-(5,6,7,8-tetrahydro-5,5,8,8-tetramethyl-2-naphthyl)-propenyl]-benzoyl]-β-D-glucopyranuronat

Die Verbindungen der Formel I können als Heilmittel, z. B. in Form pharmazeutischer Präparate, Anwendung finden. Die zur systemischen Anwendung dienenden Präparate können z. B. dadurch hergestellt werden, daß man eine Verbindung der Formel I als wirksamen Bestandteil nichttoxischen, inerten, an sich in solchen Präparaten üblichen festen oder flüssigen Trägern zufügt. Die Mittel können enteral oder parenteral verabreicht werden. Für die enterale Applikation eignen sich z. B. Mittel in Form von Tabletten, Kapseln, Dragées Sirupen, Suspensionen, Lösungen und Suppositorien. Für die parenterale Applikation sind Mittel in Form von Infusions- oder Injektions-Lösungen geeignet.

Die Dosierungen, in denen die Verfahrensprodukte verabreicht werden, können je nach Anwendungsart und Anwendungsweg sowie nach den Bedürfnissen der Patienten variieren.

Die Verfahrensprodukte können in Mengen von ca. 0,01 bis ca. 5 mg täglich in einer oder mehreren Dosierungen verabreicht werden. Eine bevorzugte Darreichungsform sind Kapseln mit einem Gehalt von ca. 0,1 mg bis ca. 1,0 mg Wirkstoff.

Die Präparate können inerte oder auch pharmakodynamisch aktive Zusätze enthalten. Tabletten oder Granula z. B. können eine Reihe von Bindemitteln, Füllstoffen, Trägersubstanzen oder Verdünnungsmitteln enthalten. Flüssige Präparate können beispielsweise in Form einer sterilen, mit Wasser mischbaren Lösung vorliegen. Kapseln können neben dem Wirkstoff zusätzlich ein Füllmaterial oder Verdickungsmittel enthalten. Des weiteren können geschmacksverbessernde Zusätze, sowie die üblicherweise als Konservierungs-, Stabilisierungs-, Feuchthalte- und Emulgiermittel verwendeten Stoffe, ferner auch Salze zur Veränderung des osmotischen Druckes, Puffer und andere Zusätze vorhanden sein.

Die vorstehend erwähnten Trägersubstanzen und Verdünnungsmittel können aus organischen oder anorganischen Stoffen, z. B. aus Wasser, Gelatine, Milchzucker, Stärke, Magnesiumstearat, Talkum, Gummi arabicum, Polyalkylenglykolen und dgl. bestehen. Voraussetzung ist, daß alle bei der

Herstellung der Präparate verwendeten Hilfsstoffe untoxisch sind.

Zur topischen Anwendung werden die Verfahrensprodukte zweckmäßig in Form von Salben, Tinkturen, Cremen, Lösungen, Lotionen, Sprays, Suspensionen und dgl. verwendet. Bevorzugt sind Salben und Creme sowie Lösungen. Diese zur topischen Anwendung bestimmten Präparate können dadurch hergestellt werden, daß man die Verfahrensprodukte als wirksamen Bestandteil nichttoxischen, inerten, für topische Behandlung geeigneten, an sich in solchen Präparaten üblichen festen oder flüssigen Trägern zumischt.

Für die topische Anwendung sind zweckmäßig ca. 0,01 bis ca. 0,3%ige, vorzugsweise 0,02 bis 0,1%ige Lösungen sowie ca. 0,05 bis ca. 5%ige, vorzugsweise ca. 0,05 bis ca. 1%ige, Salben oder Creme geeignet.

Den Präparaten kann gegebenenfalls ein Antioxydationsmittel, z. B. Tocopherol, N-Methyl-$\gamma$-tocopheramin sowie butyliertes Hydroxyanisol oder butyliertes Hydroxytoluol beigemischt sein.

Die folgenden Beispiele sollen die Erfindung weiter erläutern.

## Beispiel 1

Eine Lösung von 3,4 g Methyl-2,3,4-tri-O-acetyl-$\alpha$-D-glucopyranuronat in 40 ml Pyridin wurde bei 0° mit einer Lösung von p-[(E)-2-(5,6,7,8-Tetrahydro-5,5,8,8-tetramethyl-2-naphthyl)-propenyl]-benzoylchlorid in 70 ml Äther umgesetzt. Das Gemisch wurde bei Raumtemperatur über Nacht gerührt, zur Trockene eingedampft und der Rückstand auf Silicagel mit Hexan-Äthylacetat chromatographiert. Man erhielt Methyl-2,3,4-tri-O-acetyl-1-O-[p-[(E)-2-(5,6,7,8-tetrahydro-5,5,8,8-tetramethyl-2-naphthyl)-propenyl]-benzoyl]-D-glucopyranuronat als Gemisch der $\beta$- und $\alpha$-Anomeren (ca. 4 : 1). Schmelzpunkt 149—150° (Äthanol), $[\alpha]_D^{25} = 3°$ (c = 1 in Chloroform).

Das als Ausgangsmaterial verwendete Säurechlorid kann wie folgt hergestellt werden:

p-[(E)-2-(5,6,7,8-Tetrahydro-5,5,8,8-tetramethyl-2-naphthyl)-propenyl]-benzoesäureäthylester wird mit wäßrig-äthanolischer Kalilauge bei 55° während 18 Stunden zur entsprechenden Carbonsäure verseift und die Säure mit Thionylchlorid in Pyridin und N,N-Dimethylformamid in das Säurechlorid übergeführt.

## Beispiel 2

Ein Gemisch von 3,5 g p-[(E)-2-(5,6,7,8-Tetrahydro-5,5,8,8-tetramethyl-2-naphthyl)-propenyl]-benzoesäure, 1,05 g N,N'-Dicyclohexylcarbodiimid und 0,65 g 4-Dimethylaminopyridin in 450 ml Dichlormethan wurde 5 Stunden bei Raumtemperatur gehalten. Der Dicyclohexylharnstoff wurde abfiltriert, das Filtrat mit 1,7 g Methyl-2,3,4-tri-O-acetyl-$\alpha$-D-glucopyranuronat, 1,05 g N,N'-Dicyclohexylcarbodiimid und 0,65 g 4-Dimethylaminopyridin versetzt und bei Raumtemperatur über Nacht stehengelassen. Das Gemisch wurde dann abfiltriert, das Filtrat eingedampft und der Rückstand chromatographiert. Man erhielt ein Gemisch der $\alpha$- und $\beta$-Anomeren (ca. 3 : 2), die durch Chromatographie an einer Silicagelsäule (Merck, Hexan-Äthylacetat) getrennt wurden. Man erhielt Methyl-2,3,4-tri-O-acetyl-1-O-[p-[(E)-2-(5,6,7,8-tetrahydro-5,5,8,8-tetramethyl-2-naphthyl)-propenyl]-benzoyl]-$\alpha$-D-glucopyranuronat, Schmelzpunkt 172° (Äthanol), $[\alpha]_D^{25} = +104,1°$ (c = 1 in Chloroform) und das entsprechende $\beta$-Anomere, Schmelzpunkt 161° (Äthanol), $[\alpha]_D^{25} = -24,6°$ (c = 1 in Chloroform).

## Beispiel 3

Eine Lösung von 100 mMol p-[(E)-2-(5,6,7,8-Tetrahydro-5,5,8,8-tetramethyl-2-naphthyl)-propenyl]-benzoylchlorid in 450 ml Äther wurde unter Rühren bei 0° im Verlauf von 1 Stunde zu einer Lösung von 19,5 g Methyl-$\alpha$-D-glucopyranosid in 500 ml Pyridin gegeben. Das Gemisch wurde weitere 4 Stunden bei 0° und 16 Stunden bei Raumtemperatur gerührt und dann zur Trockene eingedampft. Der Rückstand wurde in Äthylacetat gelöst und der Extrakt mit 3N HCl, Wasser, gesättigter wäßriger Natriumbicarbonatlösung und Wasser gewaschen. Nach Trocknen über Natriumsulfat wurde die organische Phase eingedampft und der Rückstand auf Silicagel (Hexan-Äthylacetat) chromatographiert, wobei das Methyl-2,6-bis-O-[p-[(E)-2-(5,6,7,8-tetrahydro-5,5,8,8-tetramethyl-2-naphthyl)-propenyl]-benzoyl]-$\alpha$-D-glucopyranosid, Schmelzpunkt 203—204° (Äthylacetat), $[\alpha]_D^{25} = +27,4°$ (c = 1 in Chloroform) und das Methyl-3,6-bis-O-[p-[(E)-2-(5,6,7,8-tetrahydro-5,5,8,8-tetramethyl-2-naphthyl)-propenyl]-benzoyl]-$\alpha$-D-glucopyranosid, Schmelzpunkt 128—129° (unter Zersetzung, aus Methanol), $[\alpha]_D^{25} = +135°$ (c = 1 in Chloroform) erhalten wurden.

## Beispiel 4

Eine Lösung von 3,9 g p-[(E)-2-(5,6,7,8-Tetrahydro-5,5,8,8-tetramethyl-2-naphthyl)-propenyl]-benzyl-2,3,4,6-tetra-O-acetyl-$\beta$-D-glucopyranosid in 150 ml 0,1N Natriummethoxid in Methanol wurde 30

Minuten bei Raumtemperatur gehalten, mit Amberlite IR-120 Kationenaustauscher (H-Form) neutralisiert und eingedampft. Umkristallisation aus 2-Propanol lieferte p-[(E)-2-(5,6,7,8-Tetrahydro-5,5,8,8-tetramethyl-2-naphthyl)-propenyl]-benzyl-$\beta$-D-glucopyranosid, Schmelzpunkt 156—157°, $[\alpha]_D^{25}$ = −23,8° (c=1 in Pyridin).

Herstellung des Ausgangsstoffes:

5,6,7,8-Tetrahydro-5,5,8,8-tetramethyl-naphthalin wird mit Acetylchlorid und $AlCl_3$ in Nitrobenzol zu (5,6,7,8-Tetrahydro-5,5,8,8-tetramethyl-2-naphthyl)-methylketon umgesetzt. Reduktion des Ketons mit Lithiumaluminiumhydrid in Äther liefert 5,6,7,8-Tetrahydro-$\alpha$-5,5,8,8-pentamethyl-2-naphthalin-methanol, das mit Phosphortribromid in Äther/Hexan in Gegenwart von wenig Pyridin in 2-Bromäthyl-5,6,7,8-tetrahydro-5,5,8,8-tetramethyl-naphthalin übergeführt wird. Die Bromäthylverbindung liefert mit Triphenylphosphin in Xylol unter Erwärmen während 12 Stunden das [1-(5,6,7,8-Tetrahydro-5,5,8,8-tetramethyl-2-naphthyl)-äthyl]-triphenylphosphoniumbromid, das in einer Wittig-Reaktion mit 4-Äthoxycarbonyl-benzaldehyd den p-[(E)-2-(5,6,7,8-tetrahydro-5,5,8,8-tetramethyl-2-naphthyl)-propenyl] benzoesäureäthylester, Schmelzpunkt 90—91° liefert. Der Ester wird mit $LiAlH_4$ in Äther/Tetrahydrofuran zum p-[(E)-2-(5,6,7,8-Tetrahydro-5,5,8,8-tetramethyl-2-naphthyl)-propenyl]-benzylalkohol, Schmelzpunkt 123—124° (aus Methanol) reduziert.

Ein Gemisch von 5 g dieses Alkohols, 6,3 g 2,3,4,6-Tetra-O-acetyl-$\alpha$-D-glucopyranosylbromid und 1,5 g Quecksilber-II-cyanid wird in 250 ml Toluol 5 Stunden zum Rückfluß erhitzt. Die Salze werden abfiltriert und die Lösung mit gesättigter Natriumhydrogencarbonatlösung und Wasser gewaschen, getrocknet und eingedampft. Umkristallisation aus Methanol liefert p-[(E)-2-(5,6,7,8-Tetrahydro-5,5,8,8-tetramethyl-2-naphthyl)-propenyl]-benzyl-2,3,4,6-tetra-O-acetyl-$\beta$-D-glucopyranosid, Schmelzpunkt 134—135° (aus Methanol).

## Beispiel 5

Eine Lösung von 2,8 g Methyl-[p-[(E)-2-(5,6,7,8-tetrahydro-5,5,8,8-tetramethyl-2-naphthyl)-propenyl]-benzyl-2,3,4-tri-O-acetyl-$\beta$-D-glucopyranosid]-uronat in 100 ml methanolischem Ammoniak (bei 0° fast gesättigt) wurde 3 Tage bei Raumtemperatur stehengelassen und dann eingedampft. Umkristallisation aus Äthanol lieferte das p-[(E)-2-(5,6,7,8-Tetrahydro-5,5,8,8-tetramethyl-2-naphthyl)-propenyl]-benzyl-$\beta$-D-glucopyranosid-uronamid, Schmelzpunkt 194°, $[\alpha]_D^{25}$ = −38° (c=1 in Pyridin).

## Beispiel 6

Aus p-[(E)-2-(5,6,7,8-Tetrahydro-5,5,8,8-tetramethyl-2-naphthyl)-propenyl]-benzylalkohol und Methyl-2,3,4-tri-O-acetyl-$\alpha$-D-glucopyranosylbromid-uronat wird Methyl-[p-[(E)-2-(5,6,7,8-tetrahydro-5,5,8,8-tetramethyl-2-naphthyl)-propenyl]-benzyl-2,3,4-tri-O-acetyl-$\beta$-D-glucopyranosid]-uronat Schmelzpunkt 162—163° (Äthanol), $[\alpha]_D^{25}$ = −59,7° (c=1 in Chloroform) erhalten.

## Beispiel 7

In Analogie zu Beispiel 4 erhält man aus Methyl-[p-[(E)-2-(5,6,7,8-tetrahydro-5,5,8,8-tetramethyl-2-naphthyl)-propenyl]-benzyl-2,3,4-tri-O-acetyl-$\beta$-D glucopyranosid]-uronat das Methyl-[p-[(E)-2-(5,6,7,8-tetrahydro-5,5,8,8-tetramethyl-2-naphthyl)-propenyl]-benzyl-$\beta$-D-glucopyranosid]-uronat, Schmelzpunkt 103—104° (Zers., aus Isopropyläther), $[\alpha]_D^{25}$ = −65,6° (c=1 in Chloroform).

## Beispiel 8

1,9 g Methyl-[p-[(E)-2-(5,6,7,8-tetrahydro-5,5,8,8-tetramethyl-2-naphthyl)-propenyl]-benzyl-$\beta$-D-glucopyranosid]-uronat werden in 190 ml Methanol mit 10,8 ml 1N-methanolischer NaOH 1 Stunde zum Rückfluß erhitzt. Nach Zusatz von 2-Propanol bis zur Trübung und Kühlen kristallisierte das Natrium-p-[(E)-2-(5,6,7,8-tetrahydro-5,5,8,8-tetramethyl-2-naphthyl)-propenyl]-benzyl-$\beta$-D-glucopyranosid-uronat, Schmelzpunkt 240—242° (Zers.), $[\alpha]_D^{25}$ = −44,1° (c=1 in $H_2O$).

## Beispiel 9

Eine Lösung von 4,3 g Methyl-[p-[(E)-2-(5,6,7,8-tetrahydro-5,5,8,8-tetramethyl-2-naphthyl)-propenyl]-benzyl-$\beta$-D-glucopyranosid]-uronat wurde in 430 ml Methanol mit 24,5 ml 1N-methanolischer NaOH 1 Stunde zum Rückfluß erhitzt und dann eingedampft. Der Rückstand wurde in 100 ml Wasser

gelöst, die Lösung mit Amberlite IR-120 ($H^+$)-Harz neutralisiert und gefriergetrocknet. Man erhielt p-[(E)-2-(5,6,7,8-Tetrahydro-5,5,8,8-tetramethyl-2-naphthyl)-propenyl]-benzyl-$\beta$-D-glucopyranosid-uronsäure, Schmelzpunkt 174—175° (Zers., aus Methanol-Benzol), $[\alpha]_D^{25} = -28°$ (c=1 in Pyridin).

Beispiel A

Kapseln zur oralen Verabreichung können folgende Zusammensetzung aufweisen:

| | pro Kapsel |
|---|---|
| Methyl-2,6-bis-O-[p-[(E)-2-(5,6,7,8-tetrahydro-5,5,8,8-tetramethyl-2-naphthyl)-propenyl]-benzoyl]-$\alpha$-D-glucopyranosid | 0,1 mg |
| Wachsmischung | 50,5 mg |
| Pflanzenöl | 98,9 mg |
| Trinatriumsalz der Äthylendiamintetraessigsäure | 0,5 mg |

Beispiel B

| | | |
|---|---|---|
| Methyl-2,6-bis-O-[p-[(E)-2-(5,6,7,8-tetrahydro-5,5,8,8-tetramethyl-2-naphthyl)-propenyl]-benzoyl]-$\alpha$-D-glucopyranosid | | 0,1 g |
| Vaselin weiß | | 35,0 g |
| Wachs weiß | | 10,0 g |
| Paraffinöl dickflüssig | | 18,0 g |
| DEHYMULS E* | | 7,0 g |
| Benzoesäure rein | | 0,2 g |
| Wasser entsalzt | ad | 100,0 g |

Beispiel C

| | | |
|---|---|---|
| Methyl-2,6-bis-O-[p-[(E)-2-(5,6,7,8-tetrahydro-5,5,8,8-tetramethyl-2-naphthyl)-propenyl]-benzoyl]-$\alpha$-D-glucopyranosid | | 0,03 g |
| Vaselin weiß | | 35,0 g |
| Wachs weiß | | 10,0 g |
| Paraffinöl dickflüssig | | 18,0 g |
| DEHYMULS E*) | | 7,0 g |
| Benzoesäure rein | | 0,2 g |
| Wasser entsalzt | ad | 100,0 g |

*) Höhermolekularer aliphatischer Mischester; Lieferant: Deutsche Hydrierwerke

**Patentansprüche für die Vertragsstaaten: BE, CH(LI), DE, FR, GB, IT, LU, NL, SE**

1. Verbindungen der Formel

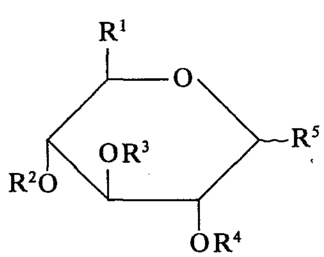

I

worin
$R^1$ ein Rest $-CH_2OH$, $-CH_2OR^6$, $-COR^7$ oder $-CONH_2$;
$R^2$ Wasserstoff oder Acetyl;
$R^3$ und $R^4$ Wasserstoff, Acetyl oder ein Rest $R^6$;
$R^5$ $C_{1-7}$-Alkoxy, ein Rest $OR^6$ oder $OR^8$;

$R^6$ ein Rest der Formel

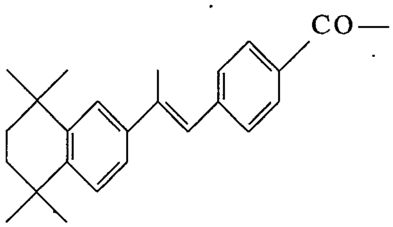

$R^7$ Hydroxy, $C_{1-7}$-alkoxy oder OM und M ein Kation; und
$R^8$ ein Rest der Formel

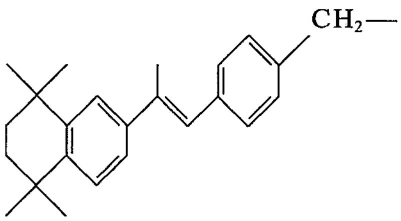

ist,
wobei $R^1$ ein Rest $CH_2OR^6$; $R^2$ Wasserstoff, und einer der Reste $R^3$ und $R^4$ Wasserstoff und der andere ein Rest $R^6$ ist, falls $R^5$ $C_{1-7}$-Alkoxy ist; und wobei $R^1$ ein Rest $-COR^7$ ist, und $R^2$, $R^3$ und $R^4$ Acetyl sind, falls $R^5$ ein Rest $OR^6$ ist; und wobei $R^1$ ein Rest $-CH_2OH$, $-COR^7$ oder $-CONH_2$ ist und $R^2$, $R^3$ und $R^4$ Wasserstoff oder Acetyl sind, falls $R^5$ ein Rest $OR^8$ ist.

2. Verbindungen gemäß Anspruch 1 der Formel

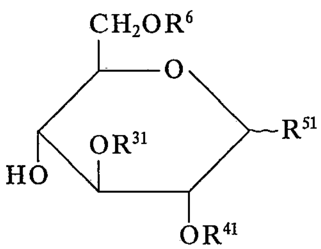

Ia

worin $R^{51}$ $C_{1-7}$-Alkoxy und einer der Reste $R^{31}$ und $R^{41}$ ein Rest $R^6$ und der andere Wasserstoff ist und $R^6$ die in Anspruch 1 angegebene Bedeutung besitzt.

3. Verbindungen gemäß Anspruch 1 der Formel

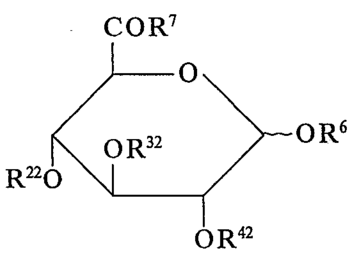

Ib

worin $R^{22}$, $R^{32}$ und $R^{42}$ Acetyl sind und $R^6$ und $R^7$ die in Anspruch 1 angegebene Bedeutung besitzen.

4. Verbindungen gemäß Anspruch 1 der Formel

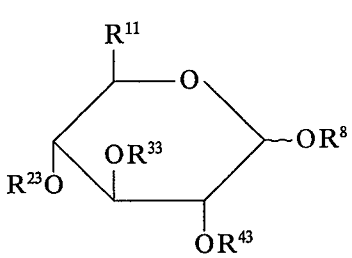

Ic

worin $R^{11}$ ein Rest $CH_2OH$, $COR^7$ oder $CONH_2$; und $R^{23}$, $R^{33}$, $R^{43}$ Wasserstoff oder Acetyl sind und $R^8$ die in Anspruch 1 angegebene Bedeutung besitzt.

5. Verbindungen gemäß Anspruch 2, worin $R^{51}$ Methoxy ist.

6. Verbindungen gemäß Anspruch 3, worin $R^7$ Methoxy ist.

7. Verbindungen gemäß Anspruch 4, worin $R^{11}$ ein Rest $-CONH_2$ oder $-COONa$ ist.

8. Methyl-2,6-bis-O-[p-[(E)-2-(5,6,7,8-tetrahydro-5,5,8,8-tetramethyl-2-naphthyl)-propenyl]-benzoyl]-α-D-glucopyranosid und Methyl-3,6-bis-O-[p-[(E)-2-(5,6,7,8-tetrahydro-5,5,8,8-tetramethyl-2-naphthyl)-propenyl]-benzoyl]-α-D-glucopyranosid gemäß Anspruch 2.

9. Methyl-2,3,4-tri-O-acetyl-1-O-[p-[(E)-2-(5,6,7,8-tetrahydro-5,5,8,8-tetramethyl-2-naphthyl)-propenyl]-benzoyl]-α-D-glucopyranuronat und Methyl-2,3,4-tri-O-acetyl-1-O-[p-[(E)-2-(5,6,7,8-tetrahydro-5,5,8,8-tetramethyl-2-naphthyl)-propenyl]-benzoyl]-β-D-glucopyranuronat, gemäß Anspruch 3.

10. p-[(E)-2-(6,7,8-Tetrahydro-5,5,8,8-tetramethyl-2-naphthyl)-propenyl]-benzyl-β-D-glucopyranosid, p-[(E)-2-(5,6,7,8-Tetrahydro-5,5,8,8-tetramethyl-2-naphthyl)-propenyl]-benzyl-β-D-glucopyranosid-uronamid, Methyl-[p-[(E)-2-(5,6,7,8-tetrahydro-5,5,8,8-tetramethyl-2-naphthyl)-propenyl]-benzyl-2,3,4-tri-O-acetyl-β-D-glucopyranosid]-uronat, Methyl-[p-[(E)-2-(5,6,7,8-tetrahydro-5,5,8,8-tetramethyl-2-naphthyl)-propenyl]-benzyl-β-D-glucopyranosid]-uronat, Natrium-p-[(E)-2-(5,6,7,8-tetrahydro-5,5,8,8-tetramethyl-2-naphthyl)-propenyl]-benzyl-β-D-glucopyranosid-uronat und p-[(E)-2-(5,6,7,8-Tetrahydro-5,5,8,8-tetramethyl-2-naphthyl)-propenyl]benzyl-β-D-glucopyranosiduronsäure gemäß Anspruch 4.

11. Verbindungen der Formel I gemäß einem der Ansprüche 1 – 10 als Heilmittel.

12. Verbindungen der Formel I gemäß einem der Ansprüche 1 – 10 bei der Behandlung von Neoplasmen, Akne, Psoriasis oder Dermatosen.

13. Pharmazeutische Präparate, enthaltend eine Verbindung der Formel I.

14. Verfahren zur Herstellung von Verbindungen der Formel I, dadurch gekennzeichnet, daß man

a) eine Verbindung der Formel

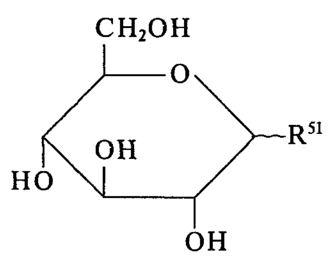

II

oder

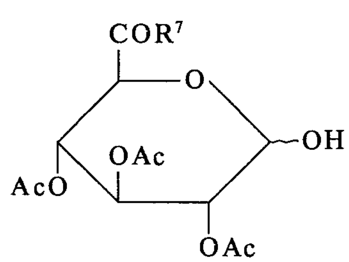

II-1

worin $R^{51}$ $C_{1-7}$-Alkoxy und $R^7$ dasselbe wie in Anspruch 1 bedeutet,
mit der Säure $R^6OH$ oder einem reaktionsfähigen Derivat davon in Gegenwart eines wasser- bzw. säurebindenden Mittels umsetzt, oder

b) eine Verbindung der Formel

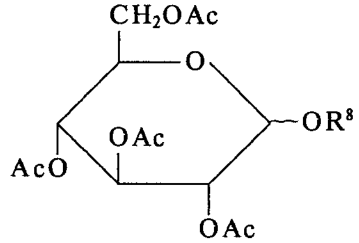

III

worin Ac Acetyl bedeutet und $R^8$ die in Anspruch 1 angegebene Bedeutung hat,
deacetyliert, oder

c) eine Verbindung der Formel

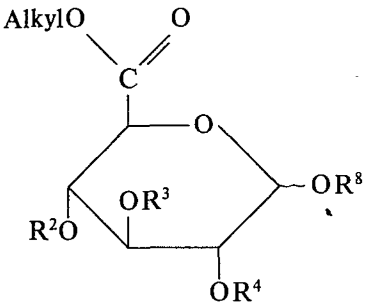

IV

mit methanolischem Ammoniak behandelt, und gewünschtenfalls eine erhaltene Verbindung der Formel I, in der $R^1$ $C_{1-7}$-Alkoxycarbonyl ist, zu einer Verbindung, in der $R^1$ Carboxyl ist, oder in ein Salz einer solchen Verbindung überführt.

**Patentansprüche für den Vertragsstaat: AT**

1. Verfahren zur Herstellung von Verbindungen der Formel

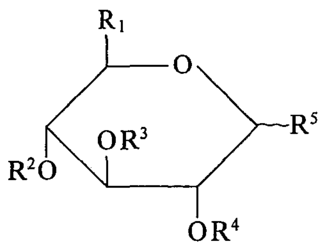

I

worin
$R^1$ ein Rest $-CH_2OH$, $-CH_2OR^6$, $-COR^7$ oder $-CONH_2$;
$R^2$ Wasserstoff oder Acetyl;
$R^3$ und $R^4$ Wasserstoff, Acetyl oder ein Rest $R^6$;
$R^5$ $C_{1-7}$-Alkoxy, ein Rest $OR^6$ oder $OR^8$;
$R^6$ ein Rest der Formel

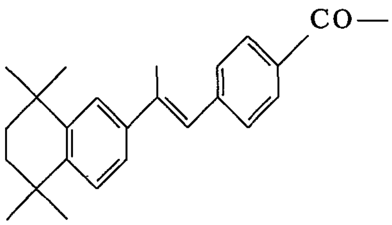

$R^7$ Hydroxy, $C_{1-7}$-Alkoxy oder OM und M ein Kation; und
$R^8$ ein Rest der Formel

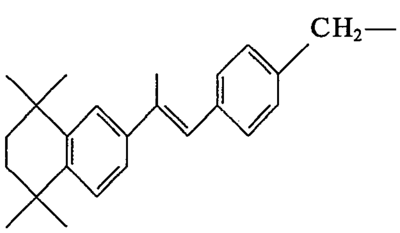

ist,
wobei $R^1$ ein Rest $CH_2OR^6$, $R^2$ Wasserstoff, und einer der Reste $R^3$ und $R^4$ Wasserstoff und der andere ein Rest $R^6$ ist, falls $R^5$ $C_{1-7}$-Alkoxy ist; und wobei $R^1$ ein Rest $-COR^7$ ist, und $R^2$, $R^3$ und $R^4$ Acetyl sind, falls $R^5$ ein Rest $OR^6$ ist; und wobei $R^1$ ein Rest $-CH_2OH$, $-COR^7$ oder $-CONH_2$ ist und $R^2$, $R^3$ und $R^4$ Wasserstoff oder Acetyl sind, falls $R^5$ ein Rest $OR^8$ ist,
dadurch gekennzeichnet, daß man

a) eine Verbindung der Formel

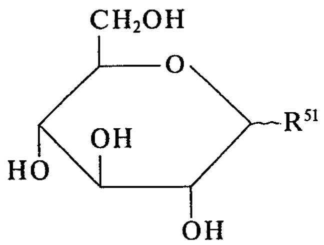

II

oder

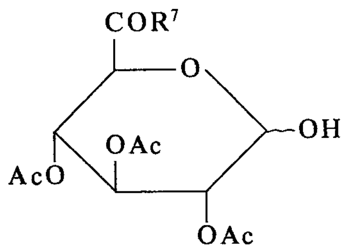

II-1

worin $R^{51}$ $C_{1-7}$-Alkoxy bedeutet, mit der Säure $R^6OH$ oder einem reaktionsfähigen Derivat davon in Gegenwart eines wasser- bzw. säurebindenden Mittels umsetzt, oder

b) eine Verbindung der Formel

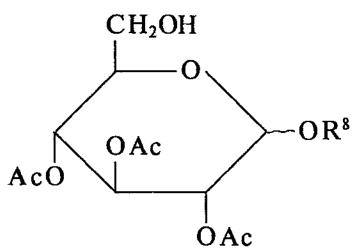

III

worin Ac Acetyl bedeutet und $R^8$ die in Anspruch 1 angegebene Bedeutung hat, deacetyliert, oder

c) eine Verbindung der Formel

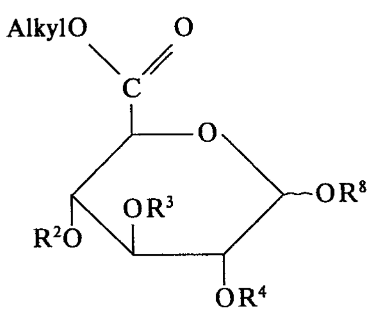

IV

mit methanolischem Ammoniak behandelt, und gewünschtenfalls eine erhaltene Verbindung der Formel I, in der $R^1$ $C_{1-7}$-Alkoxycarbonyl ist, zu einer Verbindung, in der $R^1$ Carboxyl ist, oder in ein Salz einer solchen Verbindung überführt.

2. Verfahren nach Anspruch 1, dadurch gekennzeichnet, daß man Verbindungen der Formel

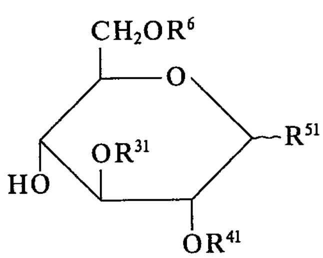

Ia

worin $R^{51}$ $C_{1-7}$-Alkoxy und einer der Reste $R^{31}$ und $R^{41}$ ein Rest $R^6$ und der andere Wasserstoff ist und $R^6$ die in Anspruch 1 angegebene Bedeutung besitzt, herstellt.

3. Verfahren nach Anspruch 1, dadurch gekennzeichnet, daß man Verbindungen der Formel

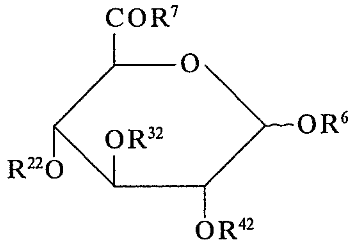

Ib

worin $R^{22}$, $R^{32}$ und $R^{42}$ Acetyl sind und $R^6$ und $R^7$ die in Anspruch 1 angegebene Bedeutung besitzen, herstellt.

4. Verfahren nach Anspruch 1, dadurch gekennzeichnet, daß man Verbindungen der Formel

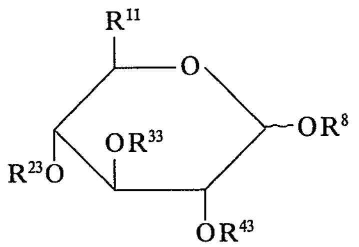

Ic

worin $R^{11}$ ein Rest $-CH_2OH$, $-COR^7$ oder $-CONH_2$; und $R^{23}$, $R^{33}$, $R^{43}$ Wasserstoff oder Acetyl sind und $R^8$ die in Anspruch 1 angegebene Bedeutung besitzt, herstellt.

5. Verfahren nach Anspruch 2, dadurch gekennzeichnet, daß man Verbindungen der Formel Ia herstellt, worin $R^{51}$ Methoxy ist.

6. Verfahren nach Anspruch 3, dadurch gekennzeichnet, daß man Verbindungen der Formel Ib herstellt, worin $R^7$ Methoxy ist.

7. Verfahren nach Anspruch 4, dadurch gekennzeichnet, daß man Verbindungen der Formel Ic herstellt, in denen $R^{11}$ ein Rest $-CONH_2$ oder $-COONa$ ist.

8. Verfahren nach Anspruch 1, dadurch gekennzeichnet, daß man Methyl-2,6-bis-O-[p-[(E)-2-(5,6,7,8-tetrahydro-5,5,8,8-tetramethyl-2-naphthyl)-propenyl]-benzoyl]-α-D-glucopyranosid und Methyl-3,6-bis-O-[p-[(E)-2-(5,6,7,8-tetrahydro-5,5,8,8-tetramethyl-2-naphthyl)-propenyl]-benzoyl]-α-D-glucopyranosid herstellt.

9. Verfahren nach Anspruch 1, dadurch gekennzeichnet, daß man Methyl-2,3,4-tri-O-acetyl-1-O-[p-[(E)-2-(5,6,7,8-tetrahydro-5,5,8,8-tetramethyl-2-naphthyl)-propenyl]-benzoyl]-α-D-glucopyranuronat und Methyl-2,3,4-tri-O-acetyl-1-O-[p-[(E)-2-(5,6,7,8-tetrahydro-5,5,8,8-tetramethyl-2-naphthyl)-propenyl]-benzoyl-β-D-glucopyranuronat herstellt.

10. Verfahren nach Anspruch 1, dadurch gekennzeichnet, daß man p-[(E)-2-(5,6,7,8-Tetrahydro-5,5,8,8-tetramethyl-2-naphthyl)-propenyl]-benzyl-β-D-glucopyranosid, p-[(E)-2-(5,6,7,8-Tetrahydro-5,5,8,8-tetramethyl-2-naphthyl)-propenyl]-benzyl-β-D-glucopyranosid-uronamid, Methyl-[p-[(E)-2-(5,6,7,8-tetrahydro-5,5,8,8-tetramethyl-2-naphthyl)-propenyl]-benzyl-2,3,4-tri-O-acetyl-β-D-glucopyranosid]-uronat, Methyl-[p-[(E)-2-(5,6,7,8-tetrahydro-5,5,8,8-tetramethyl-2-naphthyl)-propenyl]-benzyl-β-D-glucopyranosid]-uronat, Natrium-p-[(E)-2-(5,6,7,8-tetrahydro-5,5,8,8-tetramethyl-2-naphthyl)-propenyl]-benzyl-β-D-glucopyranosid-uronat und p-[(E)-2-(5,6,7,8-Tetrahydro-5,5,8,8-tetramethyl-2-naphthyl)-propenyl]-benzyl-β-D-glucopyranosid-uronsäure herstellt.

**Claims for the Contracting States BE, CH, DE, FR, GB, IT, LI, LU, NL, SE**

1. Compounds of the formula

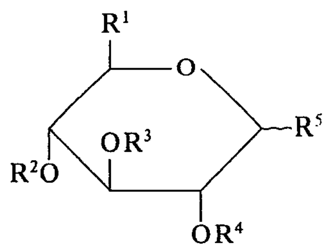

I

wherein
$R^1$ is a residue $-CH_2OH$, $-CH_2OR^6$, $-COR^7$ or $-CONH_2$;
$R^2$ is hydrogen or acetyl;
$R^3$ and $R^4$ are hydrogen, acetyl or a residue $R^6$;
$R^5$ is $C_{1-7}$-alkoxy, a residue $OR^6$ or $OR^8$;
$R^6$ is a residue of the formula

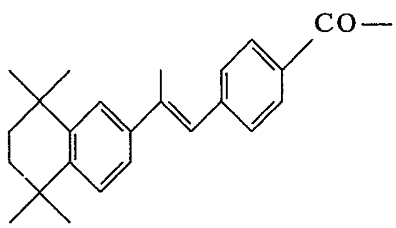

$R^7$ is hydroxy, $C_{1-7}$-alkoxy or OM and M is a cation; and
$R^8$ is a residue of the formula

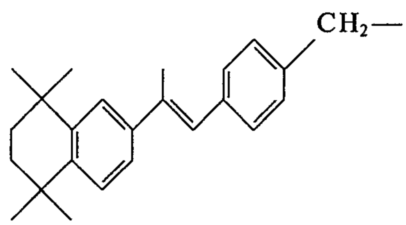

whereby $R^1$ is a residue $CH_2OR^6$; $R^2$ is hydrogen and one of the residues $R^3$ and $R^4$ is hydrogen and the other is a residue $R^6$ where $R^5$ is $C_{1-7}$-alkoxy; and whereby $R^1$ is a residue $-COR^7$ and $R^2$, $R^3$ and $R^4$ are acetyl where $R^5$ is a residue $OR^6$; and whereby $R^1$ is a residue $-CH_2OH$, $-COR^7$ or $-CONH_2$ and $R^2$, $R^3$ and $R^4$ are hydrogen or acetyl where $R^5$ is a residue $OR^8$.

2. Compounds according to claim 1 of the formula

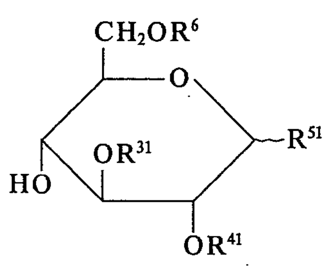

Ia

wherein $R^{51}$ is $C_{1-7}$-alkoxy and one of the residues $R^{31}$ and $R^{41}$ is a residue $R^6$ and the other is hydrogen and $R^6$ has the significance given in claim 1.

3. Compounds according to claim 1 of the formula

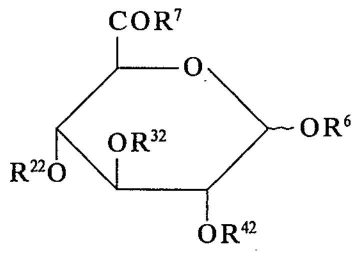

Ib

wherein $R^{22}$, $R^{32}$ and $R^{42}$ are acetyl and $R^6$ and $R^7$ have the significance given in claim 1.

4. Compounds according to claim 1 of the formula

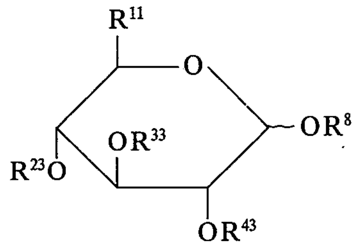

Ic

wherein $R^{11}$ is a residue $CH_2OH$, $COR^7$ or $CONH_2$; and $R^{23}$, $R^{33}$, $R^{43}$ are hydrogen or acetyl and $R^8$ has the significance given in claim 1.

5. Compounds according to claim 2, wherein $R^{51}$ is methoxy.

6. Compounds according to claim 3, wherein $R^7$ is methoxy.

7. Compounds according to claim 4, wherein $R^{11}$ is a residue $-CONH_2$ or $-COONa$.

8. Methyl 2,6-bis-O-[p-[(E)-2-(5,6,7,8-tetrahydro-5,5,8,8-tetramethyl-2-naphthyl)-propenyl]-benzoyl]-α-D-glucopyranoside and methyl 3,6-bis-O-[p-[(E)-2-(5,6,7,8-tetrahydro-5,5,8,8-tetramethyl-2-naphthyl)-propenyl]-benzoyl]-α-D-glucopyranoside according to claim 2.

9. Methyl 2,3,4-tri-O-acetyl-1-O-[p-[(E)-2-(5,6,7,8-tetrahydro-5,5,8,8-tetramethyl-2-naphthyl)-propenyl]-benzoyl]-α-D-glucopyranuronate and methyl 2,3,4-tri-O-acetyl-1-O-[p-[(E)-2-(5,6,7,8-tetrahydro-5,5,8,8-tetramethyl-2-naphthyl)-propenyl]-benzoyl]-β-D-glucopyranuronate according to claim 3.

10. p-[(E)-2-(6,7,8-Tetrahydro-5,5,8,8-tetramethyl-2-naphthyl)-propenyl]-benzyl-β-D-glucopyranoside, p-[(E)-2-(5,6,7,8-tetrahydro-5,5,8,8-tetramethyl-2-naphthyl)-propenyl]-benzyl-β-D-glucopyranoside-uronamide, methyl [p-[(E)-2-(5,6,7,8-tetrahydro-5,5,8,8-tetramethyl-2-naphthyl)-propenyl]-benzyl-2,3,4-tri-O-acetyl-β-D-glucopyranoside]-uronate, methyl [p-[(E)-2-(5,6,7,8-tetrahydro-5,5,8,8-tetramethyl-2-naphthyl)-propenyl]-benzyl-β-D-glucopyranoside]-uronate, sodium p-[(E)-2-(5,6,7,8-tetrahydro-5,5,8,8-tetramethyl-2-naphthyl)-propenyl]-benzyl-β-D-glucopyranoside-uronate and p-[(E)-2-(5,6,7,8-tetrahydro-5,5,8,8-tetramethyl-2-naphthyl)-propenyl]benzyl-β-D-glucopyranoside-uronic acid according to claim 4.

11. Compounds of formula I according to any one of claims 1—10 as medicaments.

12. Compounds of formula I according to any one of claims 1—10 in the treatment of neoplasms, acne, psoriasis or dermatoses.

13. Pharmaceutical preparations, containing a compound of formula I.

14. Process for the manufacture of compounds of formula I, characterized by

a) reacting a compound of the formula

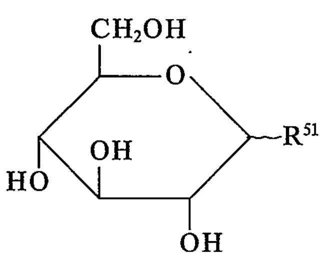

II

or

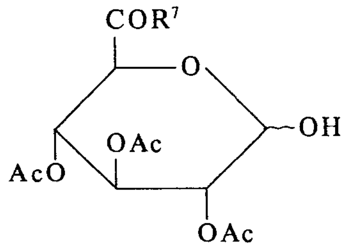

II-1

wherein $R^{51}$ signifies $C_{1-7}$-alkoxy and $R^7$ signifies the same as in claim 1, with the acid $R^6OH$ or a reactive derivative thereof in the presence of a water-binding or acid-binding agent, or

b) deacetylating a compound of the formula

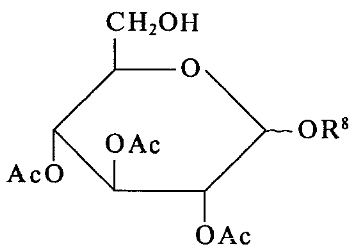

III

wherein Ac signifies acetyl and $R^8$ has the significance given in claim 1, or

c) treating a compound of the formula

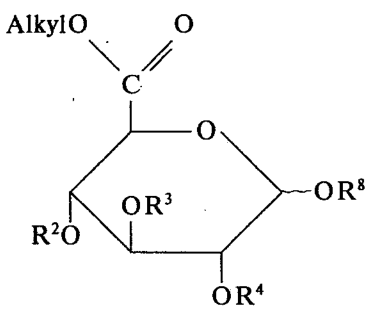

IV

with methanolic ammonia, and, if desired, converting a compound of formula I obtained in which $R^1$ is $C_{1-7}$-alkoxycarbonyl into a compound in which $R^1$ is carboxyl or into a salt of such a compound.

**Claims for the Contracting State: AT**

1. Process for the manufacture of compounds of the formula

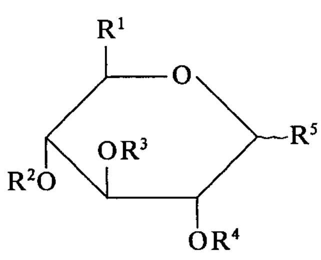

I

wherein

$R^1$ is a residue $-CH_2OH$, $-CH_2OR^6$, $-COR^7$ or $-CONH_2$;
$R^2$ is hydrogen or acetyl;
$R^3$ and $R^4$ are hydrogen, acetyl or a residue $R^6$;
$R^5$ is $C_{1-7}$-alkoxy, a residue $OR^6$ or $OR^8$;

$R^6$ is a residue of the formula

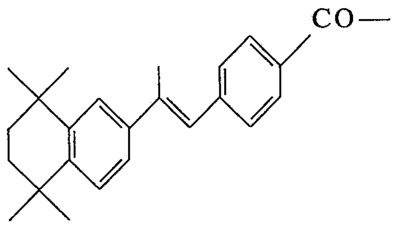

$R^7$ is hydroxy, $C_{1-7}$-alkoxy or OM and M is a cation; and
$R^8$ is a residue of the formula

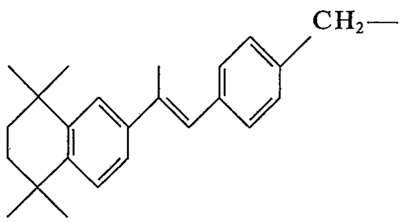

whereby $R^1$ is a residue $CH_2OR^6$, $R^2$ is hydrogen and one of the residues $R^3$ and $R^4$ is hydrogen and the other is a residue $R^6$ where $R^5$ is $C_{1-7}$-alkoxy; and whereby $R^1$ is a residue $-COR^7$ and $R^2$, $R^3$ and $R^4$ are acetyl where $R^5$ is a residue $OR^6$; and whereby $R^1$ is a residue $-CH_2OH$, $-COR^7$ or $-CONH_2$ and $R^2$, $R^3$ and $R^4$ are hydrogen or acetyl where $R^5$ is a residue $OR^8$,
characterized by

a) reacting a compound of the formula

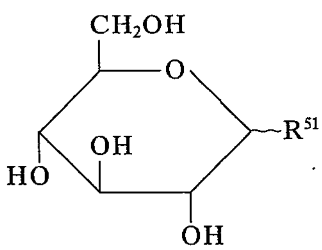

II

or

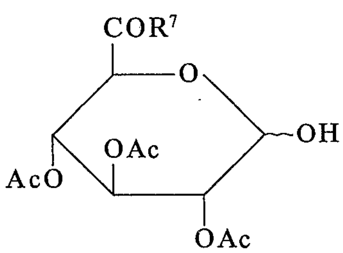

II-1

wherein $R^{51}$ signifies $C_{1-7}$-alkoxy,
with the acid $R^6OH$ or a reactive derivative thereof in the presence of a water-binding or acid-binding agent, or

b) deacetylating a compound of the formula

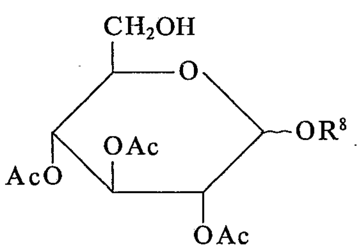

III

wherein Ac signifies acetyl and $R^8$ has the significance given in claim 1,
or

c) treating a compound of the formula

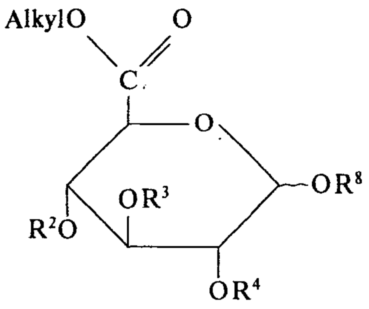

IV

with methanolic ammonia, and, if desired, converting a compound of formula I obtained in which $R^1$ is $C_{1-7}$-alkoxycarbonyl into a compound in which $R^1$ is carboxyl or into a salt of such a compound.

2. Process according to claim 1, characterized in that compounds of the formula

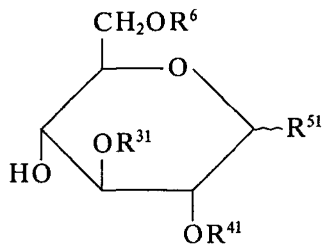

Ia

wherein $R^{51}$ is $C_{1-7}$-alkoxy and one of the residues $R^{31}$ and $R^{41}$ is a residue $R^6$ and the other is hydrogen and $R^6$ has the significance given in claim 1,
are manufactured.

3. Process according to claim 1, characterized in that compounds of the formula

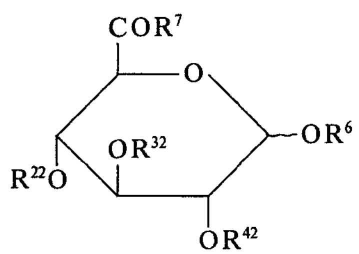

Ib

wherein $R^{22}$, $R^{32}$ and $R^{42}$ are acetyl and $R^6$ and $R^7$ have the significance given in claim 1,
are manufactured.

4. Process according to claim 1, characterized in that compounds of the formula

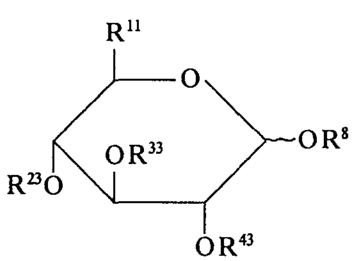

Ic

wherein $R^{11}$ is a residue $-CH_2OH$, $-COR^7$ or $-CONH_2$; and $R^{23}$, $R^{33}$, $R^{43}$ are hydrogen or acetyl and $R^8$ has the significance given in claim 1,
are manufactured.

5. Process according to claim 2, characterized in that compounds of formula Ia wherein $R^{51}$ is methoxy are manufactured.

6. Process according to claim 3, characterized in that compounds of formula Ib wherein $R^7$ is methoxy

are manufactured.

7. Process according to claim 4, characterized in that compounds of formula Ic in which $R^{11}$ is a residue $-CONH_2$ or $-COONa$ are manufactured.

8. Process according to claim 1, characterized in that methyl 2,6-bis-O-[p-[(E)-2-(5,6,7,8-tetrahydro-5,5,8,8-tetramethyl-2-naphthyl)-propenyl]-benzoyl]-α-D-glucopyranoside and methyl 3,6-bis-O-[p-[(E)-2-(5,6,7,8-tetrahydro-5,5,8,8-tetramethyl-2-naphthyl)-propenyl]-benzoyl]-α-D-glucopyranoside are manufactured.

9. Process acoording to claim 1, characterized in that methyl 2,3,4-tri-O-acetyl-1-O-[p-[(E)-2-(5,6,7,8-tetrahydro-5,5,8,8-tetramethyl-2-naphthyl)-propenyl]-benzoyl]-α-D-glucopyranuronate and methyl 2,3,4-tri-O-acetyl-1-O-[p-[(E)-2-(5,6,7,8-tetrahydro-5,5,8,8-tetramethyl-2-naphthyl)-propenyl]-benzoyl-β-D-glucopyranuronate are manufactured.

10. Process according to claim 1, characterized in that p-[(E)-2-(5,6,7,8-tetrahydro-5,5,8,8-tetramethyl-2-naphthyl)-propenyl]-benzyl-β-D-glucopyranoside, p-[(E)-2-(5,6,7,8-tetrahydro-5,5,8,8-tetramethyl-2-naphthyl)-propenyl]benzyl-β-D-glucopyranoside-uronamide, methyl [p-[(E)-2-(5,6,7,8-tetrahydro-5,5,8,8-tetramethyl-2-naphthyl)-propenyl]-benzyl-2,3,4-tri-O-acetyl-β-D-glucopyranoside]-uronate, methyl [p-[(E)-2-(5,6,7,8-tetrahydro-5,5,8,8-tetramethyl-2-naphthyl)-propenyl]-benzyl-β-D-glucopyranoside]-uronate, sodium p-[(E)-2-(5,6,7,8-tetrahydro-5,5,8,8-tetramethyl-2-naphthyl)-propenyl]-benzyl-β-D-glucopyranoside-uronate and p-[(E)-2-(5,6,7,8-tetrahydro-5,5,8,8-tetramethyl-2-naphthyl)-propenyl]-benzyl-β-D-glucopyranoside-uronic acid are manufactured.

**Revendications pour les Etats contractants BE, CH, DE, FR, GB, IT, LI, LU, NL, SE**

1. Composés de formule:

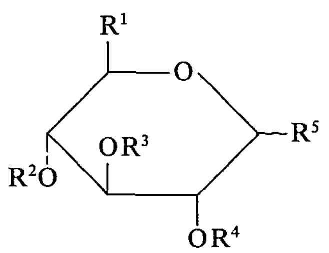

I

dans laquelle
$R^1$ représente un reste $-CH_2OH$, $-CH_2OR^6$, $-COR^7$ ou $-CONH_2$;
$R^2$ représente l'hydrogène ou un groupe acétyle;
$R^3$ et $R^4$ représentent l'hydrogène, un groupe acétyle ou un reste $R^6$;
$R^5$ représente un groupe alcoxy en $C_1-C_7$, un reste $OR^6$ ou $OR^8$;
$R^6$ est un reste de formule:

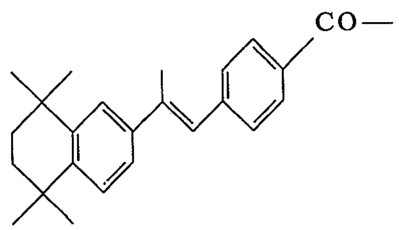

$R^7$ représente un groupe hydroxy, alcoxy en $C_1-C_7$ ou OM et M un cation; et
$R^8$ est un reste de formule:

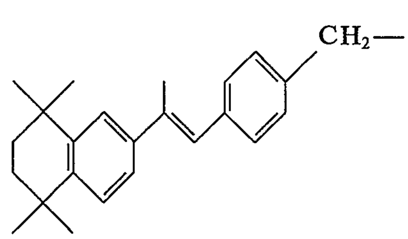

$R^1$ représentant un reste $CH_2OR^6$; $R^2$ l'hydrogène ou l'un des symboles $R^3$ et $R^4$ l'hydrogène et l'autre un reste $R^6$ lorsque $R^5$ représente un groupe alcoxy en $C_1-C_7$; et $R^1$ représente un reste $-COR^7$, et $R^2$, $R^3$ et

$R^4$ des groupes acétyles lorsque $R^5$ représente un reste $OR^6$, et $R^1$ représente un reste $-CH_2OH$, $-COR^7$ ou $-CONH_2$ et $R^2$, $R^3$ et $R^4$ représentent l'hydrogène ou des groupes acétyles lorsque $R^5$ représente un reste $OR^8$.

2. Composés selon la revendication 1 de formule:

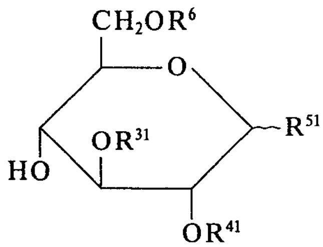

Ia

dans laquelle $R^{51}$ représente un groupe alcoxy en $C_1-C_7$ et l'un des symboles $R^{31}$ et $R^{41}$ un reste $R^6$ et l'autre l'hydrogène, $R^6$ ayant la signification indiquée dans la revendication 1.

3. Composés selon la revendication 1 de formule:

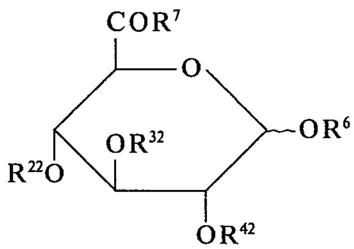

Ib

dans laquelle $R^{22}$, $R^{32}$ et $R^{42}$ représentent des groupes acétyles et $R^6$ et $R^7$ ont les significations indiquées dans la revendication 1.

4. Composés selon la revendication 1 de formule:

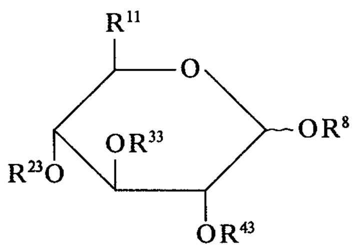

Ic

dans laquelle $R^{11}$ représente un reste $-CH_2OH$, $-COR^7$ ou $-CONH_2$; et $R^{23}$, $R^{33}$, $R^{43}$ représentent l'hydrogène ou des groupes acétyles, et $R^8$ a les significations indiquées dans la revendication 1.

5. Composés selon la revendication 2, dans lesquels $R^{51}$ représente un groupe méthoxy.

6. Composés selon la revendication 3, dans lesquels $R^7$ représente un groupe méthoxy.

7. Composés selon la revendication 4, dans lesquels $R^{11}$ représente un reste $-CONH_2$ ou $-COONa$.

8. Le méthyl-2,6-bis-O-[p-[(E)-2-(5,6,7,8-tétrahydro-5,5,8,8-tétraméthyl-2-naphthyl)-propényl]-benzoyl]-α-D-glucopyrannoside et le méthyl-3,6-bis-O-[p-[(E)-2-(5,6,7,8-tétrahydro-5,5,8,8-tétraméthyl-2-naphtyl)-propényl]-benzoyl]-α-D-glucopyrannoside selon la revendication 2.

9. Leméthyl-2,3,4-tri-O-acétyl-1-O-[p-[(E)-2-(5,6,7,8-tétrahydro-5,5,8,8-tétraméthyl-2-naphtyl)-propényl]-benzoyl]-α-D-glucopyrannuronate et le méthyl-2,3,4-tri-O-acétyl-1-O-[p-[(E)-2-(5,6,7,8-tétrahydro-5,5,8,8-tétraméthyl-2-naphtyl)-propényl]-benzoyl]-β-D-glucopyrannuronate selon la revendication 3.

10. Le p-[(E)-2-(6,7,8-tétrahydro-5,5,8,8-tétraméthyl-2-naphtyl)-propényl]-benzyl-β-D-glucopyrannoside, le p-[(E)-2-(5,6,7,8-tétrahydro-5,5,8,8-tétraméthyl-2-naphtyl)-propényl]-benzyl-β-D-glucopyrannosideuronamide, le méthyl-[p-[(E)-2-(5,6,7,8-tétrahydro-5,5,8,8-tétraméthyl-2-naphtyl)-propényl]-benzyl-2,3,4-tri-O-acétyl-β-D-glucopyrannoside]-uronate, le méthyl-[p-[(E)-2-(5,6,7,8-tétrahydro-5,5,8,8-tétraméthyl-2-naphtyl)-propényl]-benzyl-β-D-glucopyrannoside]-uronate, le p-[(E)-2-(5,6,7,8-tétrahydro-5,5,8,8-tétraméthyl-2-naphtyl)-propényl]-benzyl-β-D-glucopyrannoside-uronate de sodium et l'acide p-[(E)-2-(5,6,7,8-tétrahydro-5,5,8,8-tétraméthyl-2-naphtyl)-propényl]-benzyl-β-D-glucopyrannoside-uronique selon la revendication 4.

11. Composés de formule I selon l'une des revendications 1 à 10 en tant que médicaments.

12. Composés de formule I selon l'une des revendications 1 à 10 pour le traitement des néoplasmes,

de l'acné, du psoriasis ou des dermatoses.

13. Compositions pharmaceutiques contenant un composé de formule I.

14. Procédé de préparation des composés de formule I, caractérisé en ce que:

a) on fait réagir un composé de formule:

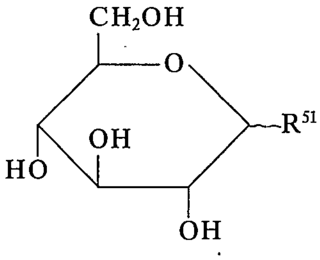

II

ou

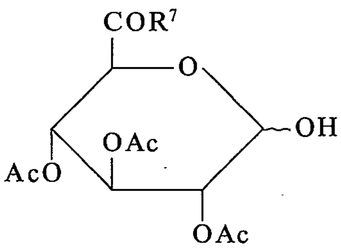

II-1

dans laquelle $R^{51}$ représente un groupe alcoxy en $C_1-C_7$ et $R^7$ a la signification indiquée dans la revendication 1,
avec l'acide $R^6OH$ ou un dérivé réactif de cet acide en présence d'un agent déshydratant ou fixant les acides respectivement, ou bien

b) on soumet à désacétylation un composé de formule:

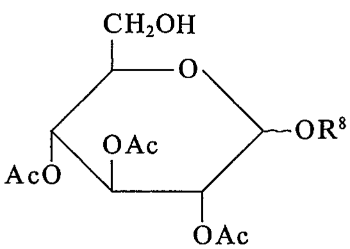

III

dans laquelle Ac représente le groupe acétyle et $R^8$ a la signification indiquée dans la revendication 1, ou bien

c) on traite un composé de formule:

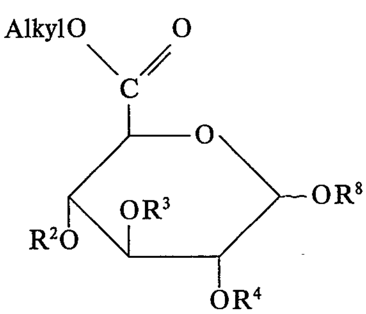

IV

par l'ammoniac méthanolique, et si on le désire on convertit un composé obtenu répondant à la formule I dans laquelle $R^1$ est un groupe (alcoxy en $C_1-C_7$)-carbonyle en un composé dans lequel $R^1$ est un groupe carboxyle ou en un sel d'un tel composé.

**Revendications pour l'Etat contractant AT**

1. Procédé de préparation des composés de formule:

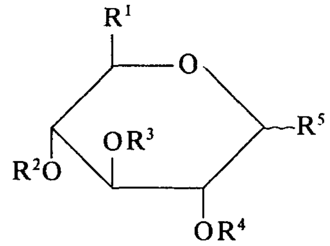

I

dans laquelle

$R^1$ représente un reste $-CH_2OH$, $-CH_2OR^6$, $-COR^7$ ou $-CONH_2$;

$R^2$ représente l'hydrogène ou un groupe acétyle;

$R^3$ et $R^4$ représentent l'hydrogène, un groupe acétyle ou un reste $R^6$;

$R^5$ représente un groupe alcoxy en $C_1-C_7$, un reste $OR^6$ ou $OR^8$;

$R^6$ est un reste de formule:

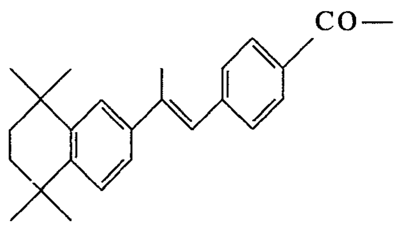

$R^7$ représente un groupe hydroxy, alcoxy en $C_1-C_7$ ou OM et M cation; et

$R^8$ est un reste de formule:

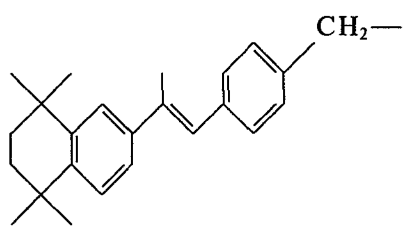

$R^1$ représentant un reste $CH_2OR^6$; $R^2$ l'hydrogène ou l'un des symboles $R^3$ et $R^4$ l'hydrogène et l'autre un reste $R^6$ lorsque $R^5$ représente un groupe alcoxy en $C_1-C_7$; et $R^1$ représente un reste $-COR^7$, et $R^2$, $R^3$ et $R^4$ des groupes acétyles lorsque $R^5$ représente un reste $OR^6$, et $R^1$ représente un reste $-CH_2OH$, $-COR^7$ ou $-CONH_2$ et $R^2$, $R^3$ et $R^4$ représentent l'hydrogène ou des groupes acétyles lorsque $R^5$ représente un reste $OR^8$, caractérisé en ce que:

a) on fait réagir un composé de formule

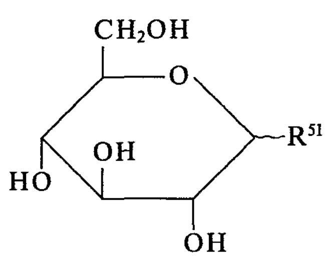

II

ou

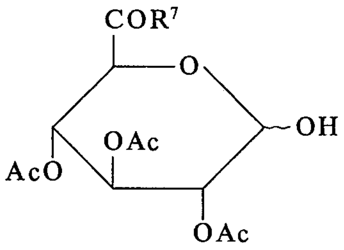

II-1

dans laquelle $R^{51}$ représente un groupe alcoxy en $C_1-C_7$ et $R^7$ a la signification indiquée dans la revendication 1, avec l'acide $R^6OH$ ou un dérivé réactif de cet acide en présence d'un agent déshydratant ou fixant les acides respectivement, ou bien

b) on soumet à désacétylation un composé de formule:

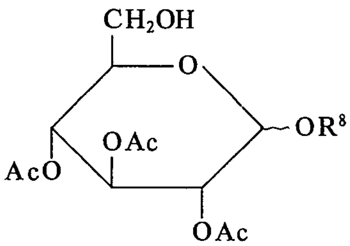

III

dans laquelle Ac représente le groupe acétyle et $R^8$ a la signification indiquée dans la revendication 1, ou bien

c) on traite un composé de formule:

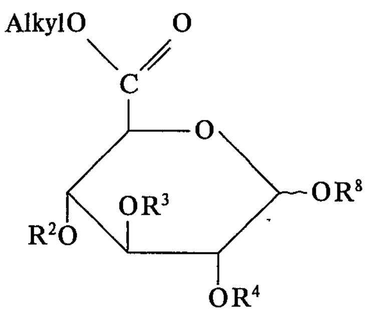

IV

par l'ammoniac méthanolique, et si on le désire on convertit un composé obtenu répondant à la formule I dans laquelle $R^1$ est un groupe (alcoxy en $C_1-C_7$)-carbonyle en un composé dans lequel $R^1$ est un groupe carboxyle ou en un sel d'un tel composé.

2. Procédé selon la revendication 1, caractérisé en ce que l'on prépare des composés de formule:

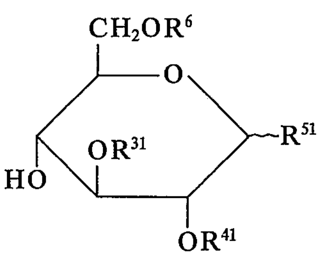

Ia

dans laquelle $R^{51}$ représente un groupe alcoxy en $C_1-C_7$ et l'un des symboles $R^{31}$ et $R^{41}$ représente un reste $R^6$ et l'autre l'hydrogène, $R^6$ ayant les significations indiquées dans la revendication 1.

3. Procédé selon la revendication 1, caractérisé en ce que l'on prépare des composés de formule:

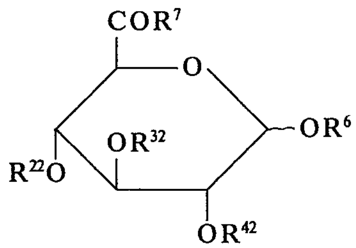

Ib

dans laquelle $R^{22}$, $R^{32}$ et $R^{42}$ sont des groupes acétyles et $R^6$ et $R^7$ ont les significations indiquées dans la revendication 1.

4. Procédé selon la revendication 1, caractérisé en ce que l'on prépare des composés de formule:

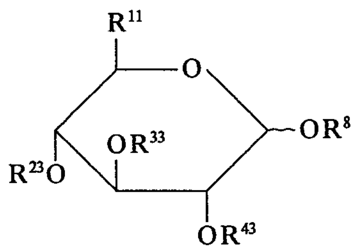

Ic

dans laquelle $R^{11}$ représente un groupe $-CH_2OH$, $-COR^7$ ou $-CONH_2$; et $R^{23}$, $R^{33}$ et $R^{43}$ représentent l'hydrogène ou des groupes acétyles, $R^8$ ayant les significations indiquées dans la revendication 1.

5. Procédé selon la revendication 2, caractérisé en ce que l'on prépare des composés de formule Ia dans laquelle $R^{51}$ est un groupe méthoxy.

6. Procédé selon la revendication 3, caractérisé en ce que l'on prépare des composés de formule Ib dans laquelle $R^7$ est un groupe méthoxy.

7. Procédé selon la revendication 4, caractérisé en ce que l'on prépare des composés de formule Ic dans lesquels $R^{11}$ représente un reste $-CONH_2$ ou $-COONa$.

8. Procédé selon la revendication 1, caractérisé en ce que l'on prépare le méthyl-2,6-bis-O-[p-[(E)-2-(5,6,7,8-tétrahydro-5,5,8,8-tétraméthyl-2-naphtyl)-propényl]-benzoyl]-α-D-glucopyrannoside et le méthyl-3,6-bis-O-[p-[(E)-2-(5,6,7,8-tétrahydro-5,5,8,8-tétraméthyl-2-naphtyl)-propényl]-benzoyl]-α-D-glucopyrannoside.

9. Procédé selon la revendication 1, caractérisé en ce que l'on prépare le méthyl-2,3,4-tri-O-acétyl-1-O-[p-[(E)-2-(5,6,7,8-tétrahydro-5,5,8,8-tétraméthyl-2-naphtyl)-propényl]-benzoyl]-α-D-glucopyrannuronate et le méthyl-2,3,4-tri-O-acétyl-1-O-[p-[(E)-2-(5,6,7,8-tétrahydro-5,5,8,8-tétraméthyl-2-naphtyl)-propényl]-benzoyl]-β-D-glucopyrannuronate.

10. Procédé selon la revendication 1, caractérisé en ce que l'on prépare le p-[(E)-2-(6,7,8-tétrahydro-5,5,8,8-tétraméthyl-2-naphthyl)-propényl]-benzyl-β-D-glucopyrannoside, le p-[(E)-2-(5,6,7,8-tétrahydro-5,5,8,8-tétraméthyl-2-naphthyl)-propényl]-benzyl-β-D-glucopyrannosideuronamide, le méthyl-[p-[(E)-2-(5,6,7,8-tétraméthyl-2-naphtyl)propényl]-benzyl-2,3,4-tri-O-acétyl-β-D-glucopyrannoside]-uronate, le méthyl-[p-[(E)-2-(5,6,7,8-tétrahydro-5,5,8,8-tétraméthyl-2-naphtyl)-propényl]-benzyl-β-D-glucopyrannoside]-uronate, le p-[(E)-2-(5,6,7,8-tétrahydro-5,5,8,8-tétraméthyl-2-naphtyl)-propényl]-benzyl-β-D-glucopyrannoside-uronate de sodium et l'acide p-[(E)-2-(5,6,7,8-tétrahydro-5,5,8,8-tétraméthyl-2-naphtyl)-propényl]-benzyl-β-D-glucopyrannoside-uronique.